(19) Europäisches Patentamt

European Patent Office

Office européen des brevets

(11) Veröffentlichungsnummer : **0 110 412**
**B1**

(12) **EUROPÄISCHE PATENTSCHRIFT**

(45) Veröffentlichungstag der Patentschrift :
28.05.86

(21) Anmeldenummer : 83112079.5

(22) Anmeldetag : 01.12.83

(51) Int. Cl.⁴ : **C 07 D249/08, B 01 J 12/00**

(54) **Verfahren zur Herstellung von 1,2,4-Triazol.**

(30) Priorität : 07.12.82 DE 3245110

(43) Veröffentlichungstag der Anmeldung :
13.06.84 Patentblatt 84/24

(45) Bekanntmachung des Hinweises auf die Patenterteilung : 28.05.86 Patentblatt 86/22

(84) Benannte Vertragsstaaten :
BE CH DE FR GB IT LI NL

(56) Entgegenhaltungen :
EP-A- 0 003 500
EP-A- 0 030 209
CH-A- 214 894

(73) Patentinhaber : BASF Aktiengesellschaft
Carl-Bosch-Strasse 38
D-6700 Ludwigshafen (DE)

(72) Erfinder : Dockner, Toni, Dr.
Grossgasse 6
D-6701 Meckenheim (DE)
Erfinder : Krug, Herbert
49 Mussbacher Strasse
D-6700 Ludwigshafen (DE)
Erfinder : Sann, Werner
Otto-Dill-Strasse 3
D-6710 Frankenthal (DE)

## Beschreibung

Die vorliegende Erfindung betrifft ein Verfahren zur Herstellung von 1,2,4-Triazol durch Umsetzung von Hydrazin und Formamid bei erhöhten Temperaturen in einem mit einem Mineralöl gefüllten Reaktor.

Es ist aus J. Amer. Chem. Soc. 77, 622 (1955) bekannt, Hydrazin-Hydrat (1 Mol) und Formamid (2 Mol) in einer ersten Reaktionsstufe zu Diformylhydrazin umzusetzen und dieses dann in einer zweiten Reaktionsstufe in flüssigem Ammoniak in einem geschlossenen System unter Druck durch 24-stündiges Erhitzen auf 200 °C in 1,2,4-Triazol überzuführen. Dabei wird 1,2,4-Triazol in einer Ausbeute von 65 % der Theorie, bezogen auf eingesetztes Hydrazin-Hydrat erhalten.

Weiterhin ist bekannt (LU-PS 61 617), 1,2,4-Triazol durch Erhitzen von & Mol Hydrazin-Hydrat mit etwa 3 Mol Formamid auf 190 bis 260 °C herzustellen. Hierzu wird das Gemisch aus Hydrazin-Hydrat und Formamid zunächst zur Bildung von Diformylhydrazin auf 70 bis 120 °C erhitzt. Anschließend wird das erhaltene Gemisch von Diformylhydrazin und Formamid auf Temperaturen bis 260 °C, vorzugsweise 180 bis 220 °C erhitzt, wobei 1,2,4-Triazol in einer Ausbeute von 80 bis 90 % gebildet wird. Zur Erzielung guter Ausbeuten ist es notwendig, die Reaktion in einer dreistufigen Kaskade durchzuführen.

Ferner ist ein Verfahren zur Herstellung von 1,2,4-Triazol bekannt, bei dem Hydrazin-Hydrat und Formamid bei 100 bis 250 °C im Molverhältnis von 1 : 2 bis 1 : 2,7 in Gegenwart von Ammoniak umgesetzt werden. Das Verfahren wird in einer dreistufigen Kaskade durchgeführt, wobei in der ersten Stufe bei 100 bis 120 °C, in der zweiten Stufe bei 180 bis 200 °C und in der dritten Stufe bei 210 bis 230 °C gearbeitet wird und die bei 110 bis 140 °C kondensierbaren flüchtigen Reaktionsprodukte der zweiten Stufe in die zweite Stufe und die bei 40 bis 80 °C kondensierbaren flüssigen Reaktionsprodukte der dritten Stufe in die erste Stufe zurückgeführt werden. Nach diesem Verfahren wird 1,2,4-Triazol in einer Ausbeute von 93 bis 94 % der Theorie und in einer Reinheit von 90 bis 98 % erhalten (Europäische Patentschrift 0 003 500).

Schließlich ist bekannt (Europäische Patentanmeldung 0 030 209), 1,2,4-Triazol durch Umsetzung von Formamid und Hydrazin im Molverhältnis von 4 : 1 bis 8 : 1 herzustellen, wobei man das Hydrazin während 2 bis 4 Stunden unterhalb der Oberfläche des auf Reaktionstemperatur erhitzten Formamids einleitet.

Wie der angegebene Stand der Technik zeigt, lassen sich mit den bekannten Verfahren gute Ausbeuten und gute Reinheit nur durch großen apparativen Aufwand und komplizierte Verfahrensmaßnahmen, wie Durchführung des Verfahrens in einer Rührkesselkaskade und Rückführung von in den einzelnen Stufen gebildeten Nebenprodukten, Anwendung eines erheblichen Überschusses an Formamid, das nach beendeter Reaktion durch aufwendige Vakuumdestillation abgetrennt werden muß, und relativ lange Reaktionszeiten, erreichen.

Es bestand daher Bedarf nach einem Verfahren zur Herstellung von 1,2,4-Triazol, bei dem die Nachteile der bekannten Verfahren vermieden werden können.

Es wurde nun ein vorteilhaftes Verfahren gefunden zur Herstellung von 1,2,4-Triazol durch Umsetzung von Hydrazin und Formamid bei erhöhten Temperaturen, welches dadurch gekennzeichnet ist, daß man Formamid und Hydrazin im Molverhältnis 1,5 : 1 bis 6 : 1 gasförmig am Boden eines mit Mineralöl mit einem Siedepunkt von mindestens 150 °C gefüllten Reaktors zuführt bzw. flüssig am Boden des Reaktors zuführt und dabei das Mineralöl bei einer Temperatur im Bereich von 150 bis 400 °C hält, bei der die flüssig zugeführten Ausgangsstoffe verdampft sind, d. h. die mindestens so hoch ist wie die den sich am Boden des Reaktors einstellenden Partialdrucken der flüssig zugeführten Ausgangsstoffe Hydrazin und Formamid entsprechenden Siedepunkte der flüssig zugeführten Ausgangsstoffe Hydrazin und Formamid, und das Reaktionsproduct durch Abdestillieren gewinnt.

Nach den neuen Verfahren läßt sich 1,2,4-Triazol in einfacher Weise einstufig in einer Art Gasphasenreaktion herstellen, ohne daß es zu Abscheidungen von Zersetzungsprodukten in den Reaktoren kommt, wie sie bei den üblichen Gasphasenreaktionen infolge von Crackvorgängen leicht auftreten.

Das Hydrazin kann in wasserfreier oder in wasserhaltiger Form verwendet werden. Da wasserfreies Hydrazin schwierig herzustellen und wegen seiner geringeren Stabilität bei den erfindungsgemäß angewendeten Reaktionstemperaturen und seiner toxischen Eigenschaften schwierig zu handhaben ist, ist die Verwendung von wasserhaltigem Hydrazin bevorzugt. Der Wassergehalt des Hydrazins kann 10 bis 50 Gew. % betragen. Als besonders vorteilhaft hat sich Hydrazin-Hydrat erwiesen, das aufgrund seiner Zusammensetzung als 64 %ige Lösung von Hydrazin in Wasser angesehen werden kann.

Das Formamid für das erfindungsgemäße Verfahren kann in handelsüblicher Reinheit eingesetzt werden.

Das Molverhältnis von Formamid zu Hydrazin beträgt wie angegeben 1,5 : 1 bis 6 : 1, vorzugsweise 2 : 1 bis 4 : 1, insbesondere 2,2 : 1 bis 3 : 1.

Das erfindungsgemäße Verfahren wird in der Weise durchgeführt, daß man das Hydrazin und Formamid gasförmig am Boden eines mit Mineralöl mit einem Siedepunkt von mindestens 150 °C, vorzugsweise mindestens 200 °C, insbesondere mindestens 300 °C, bzw. mit einem über den angegebenen Temperaturen liegenden Siedebereich gefüllten Reaktors zuführt bzw. flüssig am Boden des Reaktors zuführt und dabei das Mineralöl bei einer Temperatur hält, die mindestens so hoch ist wie die den sich am Boden des Reaktors einstellenden Partialdrucken der flüssig zugeführten Ausgangsstoffe Hydrazin und Formamid entsprechenden Siedepunkte der flüssig zugeführten Ausgangsstoffe Hydrazin und Formamid, und das Reaktionsprodukt gasförmig am Kopf des Reaktors abzieht. Als Mineralöl kommen beispielsweise hochsiedende Kohlenwasserstoffe oder Kohlenwasserstoff-Fraktionen wie Gasöl, Heizöl, geschmolzenes Paraffinwachs, ein aromatisches Kohlenwasserstofföl in Betracht. Vorteilhaft wird Vakuumgasöl mit einem Siedepunkt von mindestens 350 °C, insbesondere mit einem Siedebereich zwischen 350 °C und 550 °C verwendet.

In einer bevorzugten Ausführungsform des Verfahrens wird zusätzlich zu Formamid und Hydrazin für die Umsetzung Ammoniak eingesetzt. Als Ammoniak kann für das erfindungsgemäße Verfahren handelsübliches Ammoniak eingesetzt werden. Vorzugsweise wird das Ammoniak gasförmig am Boden des Reaktors zugeführt. Im allgemeinen beträgt das Molverhältnis von Ammoniak zu Hydrazin 2 : 1 bis 1 : 10, vorzugsweise 1 : 1 bis 1 : 5, insbesondere 1 : 1 bis 1 : 3.

Das erfindungsgemäße Verfahren kann mit Atmosphärendruck, erhöhtem Gesamtdruck z. B. bis 10 bar oder vermindertem Gesamtdruck z. B. bis herunter zu 100 mbar durchgeführt werden. Das erfindungsgemäße Verfahren wird vorzugsweise bei 180 bis 350 °C, insbesondere 200 bis 300 °C, durchgeführt.

Als Reaktoren sind für das erfindungsgemäße Verfahren z. B. Rührkessel geeignet. Vorteilhaft werden jedoch für das neue Verfahren senkrecht angeordnete zylindrische Reaktoren wie Glockenbodenkolonnen, Blasensäulen oder Füllkörperkolonnen verwendet. Hydrazin und Formamid und bei Zugabe von Ammoniak zweckmäßig auch Ammoniak werden gasförmig am Boden des mit Mineralöl gefüllten Reaktors zugeführt bzw. flüssig am Boden des Reaktors zugeführt, wobei bei flüssiger Zuführung das Mineralöl bei einer Temperatur gehalten wird, die mindestens so hoch ist wie die den sich am Boden des Reaktors einstellenden Partialdrucken der flüssig zugeführten Ausgangsstoffe entsprechenden Siedepunkte der flüssig zugeführten Ausgangsstoffe. Es ist jedoch auch möglich, einen oder mehrere der Ausgangsstoffe außerhalb des Reaktors zu verdampfen und gasförmig am Boden des Reaktors einzuleiten und den übrigen oder die übrigen Ausgangsstoffe flüssig am Boden des Reaktors einzuleiten und dort zu verdampfen. Es kann vorteilhaft sein, den verdampften Ausgangsstoff bzw. die verdampften Ausgangsstoffe mit einem inerten Gas zu verdünnen. Geeignete inerte Gase sind z. B. Wasserdampf, Kohlendioxid und vorzugsweise Stickstoff.

Das gebildete 1,2,4-Triazol wird gasförmig am Kopf des Reaktors abgezogen und anschließend zweckmäßig kondensiert. An die Kondensation kann noch eine Reinigungsstufe, z. B. eine Destillation oder Fraktionierung oder Umkristallisation angeschlossen werden. Es ist jedoch auch möglich, das erhaltene 1,2,4-Triazol ohne weitere Reinigung für weitere Umsetzungen einzusetzen.

Das neue Verfahren kann diskontinuierlich oder kontinuierlich durchgeführt werden, wobei jedoch die kontinuierliche Arbeitsweise bevorzugt wird. Bei der kontinuierlichen Arbeitsweise kann es vorteilhaft sein, das Mineralöl kontinuierlich zuzuführen und abzuziehen, wodurch gegebenenfalls in geringen Mengen gebildete Crackprodukte mit dem Mineralöl kontinuierlich aus dem Reaktor ausgeschleust werden können. Eine Aufarbeitung und Rückführung des abgezogenen Mineralöls ist in der Regel nicht wirtschaftlich, da das Mineralöl, z. B. als Heizöl oder Vakuumgasöl in der Regel wohlfeil zur Verfügung steht. Man wird daher zweckmäßig das abgezogene, Crackprodukte enthaltende Mineralöl verbrennen und dem Reaktor frisches Mineralöl zuführen.

1,2,4-Triazol ist ein Zwischenprodukt für die Synthese von Farbstoffen, optischen Aufhellern, Alterungsschutzmitteln und Pflanzenschutzmitteln.

Das folgende Beispiel veranschaulicht die Erfindung.

Beispiel

In ein als Reaktor verwendetes senkrecht angeordnetes Doppelmantelrohr mit einer Länge von 1 300 mm und einem Durchmesser von 60 mm, das mit 3 l Glasringen (5 × 5 mm) gefüllt ist, werden 0,7 l Vakuumgasöl mit einem Siedebereich von 375 °C bis 530 °C eingefüllt. In dem Reaktor wird eine Temperatur von 200 °C eingestellt. Stündlich werden 0,79 Mol Hydrazinhydrat und 2,0 Mol Formamid getrennt aus Vorratsgefäßen über Dosierpumpen in auf 170 °C erhitzten Vorverdampfern mit jeweils 2 Mol Stickstoff vorverdampft und mittels einer Zweistoffdüse am Boden des Reaktors eingebracht. Außerdem wird stündlich 1 Mol Ammoniak am Boden des Reaktors zugeführt.

Die den Reaktor verlassenden Dämpfe werden bei 40 bis 50 °C kondensiert und anschließend fraktioniert destilliert. Man erhält stündlich 48 g 1,2,4-Triazol vom Kp. 260 °C, entsprechend einer Ausbeute von 88 % der Theorie.

**Patentansprüche**

1. Verfahren zur Herstellung von 1,2,4-Triazol durch Umsetzung von Hydrazin und Formamid bei erhöhten Temperaturen, dadurch gekennzeichnet, daß man Formamid und Hydrazin im Molverhältnis 1,5 : 1 bis 6 : 1 gasförmig am Boden eines mit Mineralöl mit einem Siedepunkt von mindestens 150 °C gefüllten Reaktors zuführt bzw. flüssig am Boden des Reaktors zuführt und dabei das Mineralöl bei einer Temperatur im Bereich von 150 bei 400 °C hält, die mindestens so hoch ist, daß die am Boden des Reaktors flüssig zugeführten Ausgangsstoffe verdampfen, und das Reaktionsprodukt durch Abdestillieren gewinnt.

2. Verfahren gemäß Anspruch 1, dadurch gekennzeichnet, daß man das Hydrazin mit Formamid in dem Mineralöl umsetzt, das Mineralöl bei Anreicherung mit Nebenprodukten erneuert und das mit Nebenprodukten angereicherte hochsiedende Mineralöl abzieht.

3. Verfahren nach Anspruch 2, dadurch gekennzeichnet, daß man das mit Nebenprodukten angereicherte Mineralöl einer Verfeuerung zuführt.

4. Verfahren nach Anspruch 1, dadurch gekennzeichnet, daß man dem Reaktor zusätzlich Ammoniak zuführt.

5. Verfahren nach Anspruch 1, dadurch gekennzeichnet, daß als Mineralöl hochsiedende Kohlenwasserstoff-Fraktionen verwendet werden.

6. Verfahren nach Anspruch 1, dadurch gekennzeichnet, daß man als Mineralöl Vakuumgasöl mit einem Siedepunkt von mindestens 350 °C verwendet.

7. Verfahren nach Anspruch 1, dadurch gekennzeichnet, daß man die Kondensationsreaktion in einem zylindrischen Reaktor durchführt.

8. Verfahren nach Anspruch 1, dadurch gekennzeichnet, daß man das Verfahren kontinuierlich durchführt.

9. Verfahren nach Anspruch 1, dadurch gekennzeichnet, daß nach das Mineralöl kontinuierlich zuführt und abzieht.

10. Verfahren nach Anspruch 1, dadurch gekennzeichnet, daß man im Reaktor eine Reaktionstemperatur aufrechterhält, die mindestens so hoch ist wie die Siedepunkte die den sich im Reaktor einstellenden Partialdrucken der Ausgangsstoffe und Reaktionsprodukte entsprechen.

11. Verfahren nach Anspruch 1, dadurch gekennzeichnet, daß mann die Umsetzung bei 180 bis 350 °C durchführt.

## Claims

1. A process for the preparation of 1,2,4-triazole by reacting hydrazine with formamide at elevated temperature, wherein formamide and hydrazine in a molar ratio of 1.5 : 1 to 6 : 1 are fed in, in gaseous form, at the bottom of a reactor charged with mineral oil having a boiling point of not less than 150 °C, or are fed in, in liquid form, at the bottom of the reactor, and the mineral oil is kept at a temperature of 150 to 400 °C which is no lower than that required to vaporize the starting materials introduced as liquids at the bottom of the reactor, and the product is distilled off.

2. A process as claimed in claim 1, wherein the hydrazine is reacted with formamide in the mineral oil, the mineral oil is renewed when it has become enriched with by-products, and the high-boiling mineral oil which is enriched with by-products is removed.

3. A process as claimed in claim 2, wherein the mineral oil enriched with by-products is fed to a combustion stage.

4. A process as claimed in claim 1, wherein ammonia is additionally fed to the reactor.

5. A process as claimed in claim 1, wherein the mineral oil used is a high-boiling hydrocarbon fraction.

6. A process as claimed in claim 1, wherein the mineral oil used is a vacuum gas oil having a boiling point of not less than 350 °C.

7. A process as claimed in claim 1, wherein the condensation reaction is carried out in a cylindrical reactor.

8. A process as claimed in claim 1, wherein operation is continuous.

9. A process as claimed in claim 1, wherein the mineral oil is continuously fed in and removed.

10. A process as claimed in claim 1, wherein the reaction temperature maintained in the reactor is no lower than the boiling points corresponding to the partial pressures, of the starting materials and products, resulting in the reactor.

11. A process as claimed in claim 1, wherein the reaction is carried out at from 180 to 350 °C.

## Revendications

1. Procédé de préparation du 1,2,4-triazole par réaction de l'hydrazine avec le formamide à des températures accrues, caractérisé en ce que le formamide et l'hydrazine sont introduits à la base d'un réacteur rempli d'une huile minérale avec un point d'ébullition égal ou supérieur à 150 °C dans un rapport molaire compris entre 1,5 : 1 et 6 : 1, soit à l'état gazeux, soit à l'état liquide, l'huile minérale étant dans ce dernier cas maintenue à une température comprise dans la gamme de 150 à 400 °C et suffisamment élevée pour provoquer la vaporisation des composés de départ introduits à l'état liquide, le produit réactionnel étant isolé par distillation.

2. Procédé suivant la revendication 1, caractérisé en ce que l'on fait réagir l'hydrazine et le formamide dans l'huile minérale, l'huile minérale à point d'ébullition élevé enrichie en sous-produits étant soutirée et remplacée par de l'huile fraîche.

3. Procédé suivant la revendication 2, caractérisé en ce que l'huile minérale enrichie en sous-produits est brûlée.

4. Procédé suivant la revendication 1, caractérisé en ce que l'on introduit en outre de l'ammoniac dans le réacteur.

5. Procédé suivant la revendication 1, caractérisé en ce que l'huile minérale est choisie parmi les coupes d'hydrocarbures à point d'ébullition élevé.

6. Procédé suivant la revendication 1, caractérisé en ce que l'on emploie comme huile minérale un « gas-oil vacuum » avec un point d'ébullition au moins égal à 350 °C.

7. Procédé suivant la revendication 1, caractérisé en ce que la réaction de condensation est réalisée dans un réacteur cylindrique.

8. Procédé suivant la revendication 1, caractérisé en ce que la réaction est réalisée en continu.

9. Procédé suivant la revendication 1, caractérisé en ce que l'addition et le soutirage de l'huile minérale sont effectués en continu.

10. Procédé suivant la revendication 1, caractérisé en ce que l'on maintient dans le réacteur une température au moins égale aux points d'ébullition des composés de départ et des produits réactionnels aux tensions partielles s'établissant dans le réacteur.

11. Procédé suivant la revendication 1, caractérisé en ce que la réaction est réalisée entre 180 et 350 °C.